# EUROPEAN PATENT APPLICATION

(11) **EP 3 545 886 A1**
(43) Date of publication of application: **02.10.2019**
(21) Application number: 16922414.4
(22) Date of filing: 30.12.2016
(51) Int. Cl.: A61B 18/12, A61B 18/14, A61B 34/20

(54) **RADIO FREQUENCY ABLATION CATHETER AND SYSTEM**

(30) Priority: 23.11.2016 CN 201611038575
(71) Applicant: Changzhou Lunghealth Medtech Company Limited, Jiangsu 213145 (CN)
(72) Inventor: LIU, Hongyi, Changzhou Jiangsu 213145 (CN); MA, Jiajun, Changzhou Jiangsu 213145 (CN)
(74) Representative: Viering, Jentschura & Partner mbB Patent- und Rechtsanwälte
(86) International application number: PCT/CN2016/113921
(87) International publication number: WO 2018/094848

(57) **Abstract**

Embodiments of the present application disclose a radiofrequency ablation catheter and a system. The radiofrequency ablation catheter includes: a catheter body; a localization sensor fixedly arranged inside the catheter body; and ablation electrodes fixedly arranged on the outer surface of the catheter body and used for generating radiofrequency currents, wherein the localization sensor generates a current signal in response to a localization magnetic field of a space where the localization sensor is located, and outputs the current signal to a control system outside the radiofrequency ablation catheter, so as to enable the control system to determine a position of the radiofrequency ablation catheter. The radiofrequency ablation catheter and system provided by the embodiments of the present application may help the ablation electrodes to be placed in the lesion positions more accurately.

## Description

### Cross-reference

The present application claims priority to China Patent Application No. 2016110385758, filed on November 23, 2016 and entitled "Radiofrequency Ablation Catheter and System", which is hereby incorporated by reference in its entirety.

### Technical Field

The present application relates to the technical field of medical instruments, and more particularly relates to a radiofrequency ablation catheter and a system.

### Background Art

Radiofrequency ablation is a minimally invasive in situ treatment technique for tumors, and its principle is that: when a high-frequency alternating current of an electrode is injected into a lesion tissue, ions in the lesion tissue change with a change of a current direction. The temperature of the lesion tissue raises, and the lesion tissue is killed at a certain temperature (generally 60 ° C), thereby finally coagulating and inactivating tumor tissues.

At present, the common radiofrequency ablation is performed by directly inserting an ablation electrode into a tumor through percutaneous puncture under the guidance of an image technology such as ultrasonic or computed tomography (CT). For lung tumors, the adoption of the percutaneous puncture through which the ablation electrode is inserted into the tumor may cause a risk of pneumothorax. Studies have shown that, the incidence of pneumothorax in the percutaneous puncture mode is 20%-40%, and pneumothorax, as a serious adverse reaction, is potentially lethal. A relatively safe method is performed by inserting an ablation catheter with an electrode into a bronchoscope, delivering it to a tumor part of the lung through a natural airway of the lung for radiofrequency ablation.

However, the bronchoscope can only reach a limited range of the bronchus and cannot cover the lesions around the lungs. Furthermore, the respiratory movement in the lung also causes the change of lesion positions, resulting in an inaccurate position of the electrode placed through the bronchoscope.

### Summary of the Invention

Multiple aspects of the present application provide a radiofrequency ablation catheter and a system, which are used for helping ablation electrodes to be placed at a lesion position more accurately.

In an embodiment of the present application, there is provided a radiofrequency ablation catheter, including:
a catheter body;
a localization sensor, fixedly arranged inside the catheter body; and
ablation electrodes, fixedly arranged on an outer surface of the catheter body and used for generating radiofrequency currents,
wherein the localization sensor generates a current signal in response to a localization magnetic field of a space where the localization sensor is located, and outputs the current signal to a control system outside the radiofrequency ablation catheter, so as to enable the control system to determine a position of the radiofrequency ablation catheter.

Optionally, a water-cooling structure for accommodating electrode cooling liquid is arranged inside the catheter body.

Optionally, the catheter body comprises therein at least two lumina along a length direction of the catheter body, wherein the localization sensor is fixedly arranged in one of the at least two lumina; and the other lumen of the at least two lumina and a gap formed by the other lumen and the lumen with the localization sensor form the water-cooling structure.

Optionally, the at least two lumina comprise: a first lumen having an axial line that is collinear with an axial line of the catheter body and a second lumen surrounding the first lumen, wherein the localization sensor is fixedly arranged in the first lumen; and the second lumen is used as a water inlet channel, and the gap between the second lumen and the first lumen is used as a water outlet channel, so as to form the water-cooling structure.

Optionally, a water-cooling pipe with openings at both ends is arranged inside the catheter body, wherein the water-cooling pipe is used as a water inlet channel, and an outer wall of the water-cooling pipe and an inner wall of the catheter body form a water outlet channel, so as to form the water-cooling structure.

Optionally, an end, away from the localization sensor, of the catheter body is provided with a water inlet pipe and a water outlet pipe, wherein the water inlet pipe is connected with an end, away from the localization sensor, of the water-cooling pipe, and the water outlet pipe is connected with an end, away from the localization sensor, of the water outlet channel.

Optionally, the localization sensor comprises: a first localization sensor fixedly arranged at a head portion of the catheter body.

Optionally, the localization sensor further includes: a second localization sensor.

Optionally, at least two ablation electrodes are provided, and are arranged on the outer surface of the catheter body along the length direction of the catheter body in a spacing manner.

Optionally, the radiofrequency ablation catheter further includes: a temperature sensor used for detecting a temperature of an ablation electrode.

Optionally, the radiofrequency ablation catheter further includes: a protective structure for preventing the localization sensor from being deformed by a force.

Optionally, the protective structure includes: a protective layer fixedly arranged on the outer surface of the localization sensor, and/or a protective cap sleeved on the head portion of the localization sensor or the head portion of the catheter body.

Optionally, the protective cap comprises: a securing portion for securing the protective cap and a protective lumen for accommodating the head portion of the localization sensor.

Optionally, a diameter of the securing portion is equal to a diameter of the protective lumen, and the securing portion is fixedly arranged on the outer surface of the catheter body; or the diameter of the securing portion is less than the diameter of the protective lumen, and the securing portion is fixedly arranged on the inner surface of the catheter body.

In an embodiment of the present application, there is further provided a radiofrequency ablation system, including:
a radiofrequency ablation catheter;
a control system configured for controlling the operation of the radiofrequency ablation catheter; and
a connector configured for connecting the radiofrequency ablation catheter with the control system,
wherein the radiofrequency ablation catheter comprises:
   a catheter body;
   a localization sensor, fixedly arranged inside the catheter body; and
   ablation electrodes, fixedly arranged on an outer surface of the catheter body and used for generating radiofrequency currents,
wherein the localization sensor generates a current signal in response to a localization magnetic field of a space where the localization sensor is located, and outputs the current signal to the control system, so as to enable the control system to determine a position of the radiofrequency ablation catheter.

In an embodiment of the present application, there is further provided an ablation catheter, including:
a catheter body;
a trocar connected and fixed at a front end of the catheter body;
an electrode catheter, arranged inside the catheter body and telescopically movable; and
a center electrode, connected and fixed at the head portion of the electrode catheter, wherein the head portion of the center electrode is fixedly provided with an umbrella electrode;
the center electrode is of a hollow structure; a localization sensor is arranged inside the center electrode; wherein the localization sensor generates a current signal in response to a localization magnetic field of a space where the localization sensor is located, and outputs the current signal to a control system outside the ablation catheter, so as to enable the control system to determine a position of the ablation catheter.

Optionally, the umbrella electrode has the same polarity as the center electrode, and a temperature sensor is further arranged inside the center electrode.

Optionally, the catheter body has a structure of tightly wrapped helical metal wires, or a structure in which braided metal wires are provided inside the catheter, or a structure in which a helical metal wire is provided inside the catheter; and the electrode catheter has a structure of tightly wrapped helical metal wires, or a structure in which braided metal wires are provided inside the catheter, or a structure in which a helical metal wire is provided inside the catheter.

In the embodiments of the present application, by arranging the localization sensor inside the radiofrequency ablation catheter, the radiofrequency ablation catheter can be accurately located to a lesion position through navigation of the localization sensor, so that the problem of inaccurate position of the radiofrequency ablation catheter placed by a bronchoscope can be solved. In addition, the localization sensor is configured to track the position of the radiofrequency ablation catheter in real time, thereby overcoming the problem that the inaccurate position of the radiofrequency ablation catheter due to change of lesion positions caused by the respiratory movement of the lung or other accidental movements.

### Brief Description of the Drawings

Drawings described herein are used for providing further understandings of the present application and constitute one part of the present application. Illustrative embodiments and descriptions of the present application are used for explaining the present application, and do not constitute an improper limitation to the present application. In the drawings:
Fig. 1 is a schematic diagram of a structure of a radiofrequency ablation catheter provided in an embodiment of the present application.
Fig. 2 is a schematic diagram of a structure of a radiofrequency ablation catheter provided in another embodiment of the present application.
Fig. 3a is a schematic diagram of a cross-section of a catheter body provided in yet another embodiment of the present application.
Fig. 3b is a schematic diagram of a structure of a radiofrequency ablation catheter provided in yet another embodiment of the present application.
Fig. 4a is a schematic diagram of a structure of a radiofrequency ablation catheter provided in yet another embodiment of the present application.
Fig. 4b is a schematic diagram of a structure of a radiofrequency ablation catheter provided in yet another embodiment of the present application.
Fig. 5 is a schematic diagram of a structure of a radiofrequency ablation catheter provided in yet another embodiment of the present application.
Fig. 6 is a schematic diagram of a structure of a radiofrequency ablation catheter provided in yet another embodiment of the present application.
Fig. 7 is a schematic diagram of a structure of a radiofrequency ablation catheter provided in yet another embodiment of the present application.
Fig. 8 is a schematic diagram of a structure of a radiofrequency ablation catheter provided in yet another embodiment of the present application.
Fig. 9 is a schematic diagram of a structure of a radiofrequency ablation system provided in yet another embodiment of the present application.
Fig. 10 is a schematic diagram of a structure of an ablation catheter provided in yet another embodiment of the present application when an umbrella electrode is in a retracted state. Fig. 11 is a schematic diagram of a structure of an ablation catheter provided in a further embodiment of the present application when an umbrella electrode is in an expanded-out state.
Fig. 12 is an overall schematic diagram of a structure of an ablation catheter provided in yet another embodiment of the present application.

### Detailed Description of the Invention

For the purpose of making objectives, technical solutions and advantages of the present application clearer, clear and complete description will be made below to the technical solutions of the present application in conjunction with specific embodiments and corresponding drawings of the present application. Obviously, the described embodiments are merely a part of the embodiments of the present application and not all the embodiments. Based on the embodiments of the present application, all other embodiments obtained by those ordinarily skilled in the art without paying creative work shall fall within the protection scope of the present application.

As an important component for implementing radiofrequency ablation, a radiofrequency ablation catheter has been widely applied to various medical practices. For example, it has been applied to practice in treating solid tumors, such as liver, kidney and lung. But, the existing radiofrequency ablation catheter itself has no localization function, thus it is required to be placed through a traditional bronchoscope, and a placement position of the radiofrequency ablation catheter is limited by a reachable range of the bronchoscope. However, the conventional bronchoscope cannot cover all parts of the bronchus and thus cannot reach the lesions around the lungs, which results in inaccuracy of the placement position of the radiofrequency ablation catheter and an unideal therapeutic effect. In addition, in consideration of the respiratory movement of the lung, the lesion position may change along with the respiratory movement of the lung, and the radiofrequency ablation catheter cannot adjust the position in real time, which also leads to the inaccuracy of the placement position of the radiofrequency ablation catheter and influences the therapeutic effect.

Aiming at the above-mentioned problems, an embodiment of the present application provides a novel radiofrequency ablation catheter. The radiofrequency ablation catheter is provided with a localization sensor in addition to an ablation electrode, so that the radiofrequency ablation catheter may be accurately located to a lesion position through navigation of the localization sensor. Furthermore, the position of the radiofrequency ablation catheter may be further tracked in real time, so as to solve the problem that the inaccurate position of the radiofrequency ablation catheter due to various reasons and enhance the therapeutic effect of the radiofrequency ablation.

With the built-in localization sensor, the radiofrequency ablation catheter provided by the embodiment of the present application is bound to be different from the traditional radiofrequency ablation catheter in structure. In addition, as the radiofrequency ablation catheter is a precision medical instrument, it is not easy to add new devices or functions. In most cases, a relatively reasonable implementation structure may be finally obtained through repeated experiments. The structure of the radiofrequency ablation catheter provided by the embodiment of the present application will be described in detail below in conjunction with the drawings.

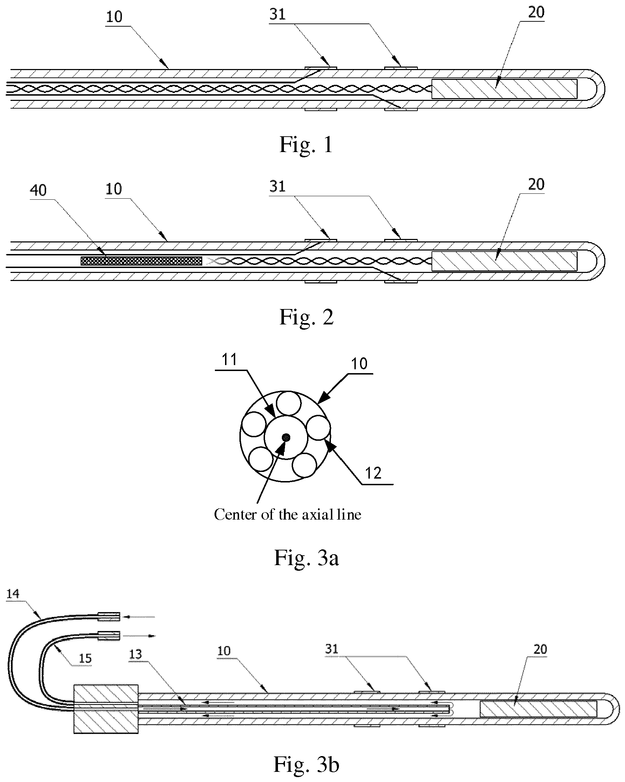

Fig. 1 is a schematic diagram of a structure of a radiofrequency ablation catheter provided by an embodiment of the present application. As shown in Fig. 1, the radiofrequency ablation catheter mainly includes: a catheter body 10, a localization sensor 20 and an ablation electrode 31.

The localization sensor 20 is fixedly arranged inside the catheter body 10, and is mainly used for locating the radiofrequency ablation catheter to accurately locate the radiofrequency ablation catheter to a lesion position. For example, the localization sensor 20 may be fixedly arranged inside the catheter body 10 by various methods such as a hot melt method and an adhesive-based adhesion method.

The localization sensor 20 in the present embodiment is based on an electromagnetic navigation principle which is different from general magnetic navigation principles. The magnetic navigation principle is mainly that: a permanent magnet in the ablation catheter is attracted or repulsed by means of an external magnetic field to affect a moving direction of the ablation catheter. The working principle of the localization sensor 20 in the present embodiment is mainly that: a current signal is generated in response to a localization magnetic field of a space where the localization sensor 20 is located and is output to a control system outside the radiofrequency ablation catheter, so as to enable the control system to determine the position of the radiofrequency ablation catheter. In detail, the localization sensor 20 is electrically connected with the control system through lead wires. The control system includes a magnetic field generator for generating a magnetic field in a localization space within a certain range. The localization sensor 20 itself is nonmagnetic, and the coil in the localization sensor 20 is used for sensing a localization magnetic field generated by the magnetic field generator. Herein, the magnetic field generator generates the localization magnetic field in the localization space within the certain range to ensure that the magnetic characteristic of each point in the localization space is unique. The coil in the localization sensor 20 generates a current signal in a variation magnetic field, and the current signal is transmitted to the control system through the lead wires of the localization sensor 20. The control system converts and analyzes the current signal transmitted by the localization sensor 20 to determine an accurate position and direction of the radiofrequency ablation catheter.

The ablation electrode 31 is fixedly arranged on the outer surface of the catheter body 10 and is mainly used for performing ablation treatment on a lesion. For example, the ablation electrode 31 may be fixedly arranged on the outer surface of the catheter body 10 by using various methods such as the hot melt method, the adhesive-based adhesion method, a swaging method and a microfluidic machining technology.

In the present embodiment, the ablation electrode 31 is used for generating a radiofrequency current. The radiofrequency current is injected into a lesion tissue, so that ions in the lesion tissue change with a change of a current direction, the temperature of the lesion tissue raises, and the lesion tissue becomes irreversibly dysfunctional at a certain temperature (generally 60 °C or greater), thereby finally coagulating and inactivating tumor tissues for therapeutic purposes.

Optionally, the above-mentioned control system further includes: a radiofrequency ablation generator used for controlling the ablation electrode 31 generates the radiofrequency current. For example, the ablation electrode 31 for generating the radiofrequency current may be selected, and the intensity of the radiofrequency current generated by the ablation electrode as well as the time that the ablation electrode generates the radiofrequency current may also be controlled. The ablation electrode 31 may specifically generate the radiofrequency current under the control of the radiofrequency ablation generator.

Optionally, the radiofrequency ablation catheter of the present embodiment may adopt a unipolar form, namely, the electrode of the catheter body 10 having only one polarity, and the electrode of the other polarity is attached to the outer surface of a human body. For example, the above-mentioned ablation electrode 31 may adopt an annular electrode, and may be made of a metal material, such as copper, stainless steel, a platinum-iridium alloy, or other conductive materials. Alternatively, the radiofrequency ablation catheter of the present embodiment may adopt a bipolar form, namely, the electrodes of the catheter body 10 having positive and negative polarities, and the electrodes of different polarities are not connected, and it is not necessary to use electrode on the outer surface of the human body.

Optionally, the ablation electrode 31 may be fixed on the outer surface of the catheter body 10 by adopting a process such as swaging, attachment, spray coating, plating and microfluidic machining.

The radiofrequency ablation catheters provided by the above-mentioned embodiments include the localization sensors in addition to the ablation electrodes. The radiofrequency ablation catheters can be accurately located to the lesion positions through the navigation by the localization sensors. In addition, the positions of the radiofrequency ablation catheters may be further tracked in real time, thereby overcoming the inaccurate positions of the radiofrequency ablation catheters due to various reasons and enhancing the therapeutic effect of the radiofrequency ablation.

Optionally, Fig. 2 is another implemented structure of the radiofrequency ablation catheter. The radiofrequency ablation catheter as shown in Fig. 2 further includes a water-cooling structure 40 in addition to the catheter body 10, the localization sensor 20 and the ablation electrode 31. The water-cooling structure 40 is arranged inside the catheter body 10 and is used for accommodating electrode cooling liquid. Optionally, the electrode cooling liquid may be clean water or normal saline, but not limited thereto. Preferably, the liquid may be at a normal temperature or at a temperature lower than a room temperature, such as cold water of 4°C. The water-cooling structure 40 can reduce the temperature of a tissue during radiofrequency ablation, so as to avoid overheating and scarring, and improve the effectiveness of the radiofrequency ablation.

The above-mentioned water-cooling structure 40 may be arranged inside and independent of the catheter body 10, and may also be integrated with the catheter body 10.

It should be noted that Fig. 2 merely illustrates that there is a structure with a water-cooling function at a corresponding position in the catheter body 10, but does not show an actual configuration or a pattern of the water-cooling structure 40.

In an implementation, the water-cooling structure 40 is integrally provided with the catheter body 10. The interior of the catheter body 10 includes: at least two lumina along a length direction of the catheter body 10. The localization sensor 20 is fixedly arranged in one of the at least two lumina. The other lumen of the at least two lumina and a gap formed by the other lumen and the lumen with the localization sensor 20 therein form the water-cooling structure 40 which is used for accommodating the electrode cooling liquid. Both ends of the other lumen are provided with openings to facilitate the circulation of the electrode cooling liquid.

For the above-mentioned at least two lumina along the length direction of the catheter body 10, the size of the diameter of each lumen and an arrangement layout among all the lumina may be not defined in the embodiments. In an implementation, the above-mentioned at least two lumina (namely, the inside of the catheter body 10) along the length direction of the catheter body 10 include: a first lumen 11 having an axial line that is collinear with the axial line of the catheter body 10 and a second lumen 12 surrounding the first lumen 11, referring to the schematic diagram of a cross-section of the catheter body 10 as shown in Fig. 3a. Herein, the localization sensor 20 is fixedly arranged in the first lumen 11, and the second lumen 12 and a gap between the second lumen 12 and the first lumen 11 form the water-cooling structure 40. Both ends of the second lumen 12 are provided with openings. Optionally, the second lumen 12 may be used as a water inlet channel, and the gap between the second lumen 12 and the first lumen 11 may be used as a water outlet channel. The number of second lumina 12 may be two or more. Preferably, the diameter of the first lumen 11 is greater than that of the second lumen 12, but not limited thereto.

Optionally, the first lumen 11 is of a semi-closed structure, that is, an end close to the head portion of the catheter body 10 is closed, so as to isolate the localization sensor 20 from the electrode cooling liquid.

In an implementation, as shown in Fig. 3b, a water-cooling pipe 13 with openings at both ends is arranged inside the catheter body 10. The water-cooling pipe 13 is used as the water inlet channel, and the outer wall of the water-cooling pipe 13 and the inner wall of the catheter body 10 form a water outlet channel, so as to constitute the water-cooling structure 40.

Further, as shown in Fig, 3b, an end, away from the localization sensor 20, of the catheter body 10 is provided with a water inlet pipe 14 and a water outlet pipe 15. Herein, the water inlet pipe 14 is connected with an end, away from the localization sensor 20, of the water-cooling pipe 13, and the water outlet pipe 15 is connected with an end, away from the localization sensor 20, of the water outlet channel.

Herein, a circulation mode of the electrode cooling liquid in the water-cooling structure shown in Fig. 3b is as shown by an arrow indicating line in Fig. 3b. The water inlet pipe 14 conveys the electrode cooling liquid through a peristaltic pump or pumps of other structures, so that the electrode cooling liquid may be delivered to the distal end (which is close to the localization sensor 20) of the catheter body 10 through the water-cooling pipe 13 and then cyclically returned to the proximal end (which is far away from the localization sensor 20) through the water outlet channel, so as to take away heat and reduce the temperature.

Optionally, in order to protect the localization sensor 20, an isolation portion may be arranged inside the catheter body 10, so as to isolate the localization sensor 20 from the electrode cooling liquid.

In the embodiments of the present application, the number of the localization sensor is not limited. One, two or more than two localization sensors may be provided.

In an implementation structure, the radiofrequency ablation catheter includes a localization sensor referred to as first localization sensor. The first localization sensor is fixedly arranged at the head portion of the catheter body 10, referring to Fig. 1. Optionally, the first localization sensor may be a 6 degree-of-freedom electromagnetic navigation sensor that satisfies a localization requirement of the radiofrequency ablation catheter.

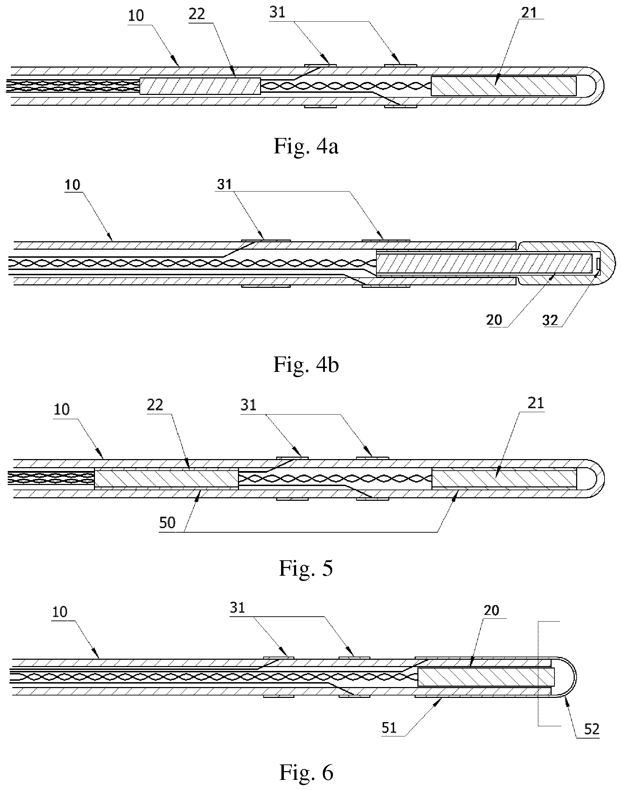

In an implementation structure, the radiofrequency ablation catheter includes two localization sensors. The use of the two localization sensors is favorable for further improving the localization accuracy. As shown in Fig. 4a, the radiofrequency ablation catheter mainly includes: a catheter main body 10, a first localization sensor 21, a second localization sensor 22 and ablation electrodes 31. The first localization sensor 21 and the second localization sensor 22 are both arranged inside the catheter body 10. The first localization sensor 21 is fixedly arranged at the head portion of the catheter body 10, and the second localization sensor 22 is spaced apart from the first localization sensor 21 by a certain distance.

Optionally, the first localization sensor 21 and the second localization sensor 22 are separately arranged on two sides of the ablation electrodes 31. Subsequent embodiments are described mainly by taking this structure for example, but not limited hereto.

In the embodiment as shown in Fig. 4a, the catheter body 10 may adopt any one of the implementation structures described in the foregoing or subsequent embodiments. Correspondingly, the ablation electrodes 31 also may adopt any one of the implementation structures described in the foregoing or subsequent embodiments. No more details will be described here for the implementation structures of the catheter body 10 and the ablation electrodes 31. In addition, in order to show the first localization sensor 21 and the second localization sensor 22 more clearly, only the related structure is shown in Fig. 4a.

In the embodiments of the present application, the number of the ablation electrode 31 is not limited. One or at least two ablation electrodes 31 may be provided. More preferably, at least two ablation electrodes 31 are provided. With at least two ablation electrodes 31, more contact areas with the lesion may be maintained to achieve a better controllable therapeutic effect. In case of at least two ablation electrodes 31, they are arranged on the outer surface of the catheter body 10 along the length direction of the catheter body in a spacing manner, as shown in Fig. 4a. The distance between adjacent ablation electrodes 31 may be the same or different.

On the basis of all the foregoing or subsequent embodiments, as shown in Fig. 4b, the radiofrequency ablation catheter includes: a catheter body 10, a localization sensor 20, ablation electrodes 31, and a temperature sensor 32 for detecting temperatures of the ablation electrodes 31. The temperature sensor 32 is mainly used for detecting the temperatures of the ablation electrodes 31 in real time and feeding the temperature back to an external control system, so that current generation power and current generation time of the ablation electrodes 31 can be controlled, and thus a treatment aim is fulfilled. It should be noted that when the radiofrequency ablation catheter is in a bipolar form, the temperature sensor 32 may be saved. For example, an ablation range may be controlled by controlling output power and time as well as by detecting impedance between electrodes with different polarities. When the radiofrequency ablation catheter is in a unipolar form, the temperature sensor 32 is required to detect the temperatures of the ablation electrodes 31. The temperature sensor 32 is generally placed at a position near to center points of the ablation electrodes 31, that is, a position (the center of an ablation region) with the highest temperature.

On the basis of all the foregoing embodiments, the radiofrequency ablation catheter further may include: a protective structure for preventing the localization sensor from deforming by a force. Under the protection of the protective structure, the localization sensor may be prevented from deforming by the force, which is favorable for improving the localization accuracy, and then helps to locate the radiofrequency ablation catheter to a lesion position more accurately. The present embodiment does not define an implementation form of the protective structure. Examples will be given below.

For example, the protective structure may be a protective layer fixedly arranged on the outer surface of the localization sensor. Preferably, in order to protect the localization sensor from be deformed by the force, the protective layer may be made of a hard material, such as, but not limited to, metal. Optionally, part or all of the localization sensors may be provided with the protective layers. The radiofrequency ablation catheter shown in Fig. 5 includes: a catheter main body 10, a first localization sensor 21, a second localization sensor 22 and ablation electrodes 31. The first localization sensor 21 and the second localization sensor 22 are separately arranged on two sides of the ablation electrodes 31. The first localization sensor 21 is fixedly arranged at the head portion of the catheter body 10, and protective layers 50 are arranged on the outer surfaces of both the first localization sensor 21 and the second localization sensor 22.

For another example, the protective structure may be a protective cap that sleeves on the head portion of the localization sensor or the head portion of the catheter body. In order to protect the localization sensor from be deformed by the force, the protective cap may be made of a hard material, such as, but not limited to, metal. Preferably, the protective cap is applicable to the localization sensor located at the head portion of the catheter body, but is not limited thereto.

Preferably, if the protective cap is made of a metal material and is electrically connected with a control system (specifically, a radiofrequency ablation generator in the control system) outside the radiofrequency ablation catheter, a radiofrequency current may be generated under the control of the control system. In the optional implementation, the protective cap may act as an ablation electrode while protecting the localization sensor.

Preferably, the surface of the protective cap is required to be smooth to allow the protective cap to move in an airway.

Preferably, the head portion of the protective cap may be of various shapes favorable for reducing resistance, such as a round shape and a streamline shape (like the shape of a water droplet), so that the whole radiofrequency ablation catheter can move in the airway flexibly.

The implementation structure of the protective cap may be divided into: a securing portion and a protective lumen. The securing portion is used for securing the protective cap. The securing portion may be fixed on the localization sensor or the catheter body. The protective lumen is used for accommodating the head portion of the localization sensor. The embodiments do not define the shape of the protective cap. Several examples will be given below.

The radiofrequency ablation catheter shown in Fig. 6 includes: a catheter body 10, a localization sensor 20 and ablation electrodes 31. The localization sensor 20 is located at the head portion of the catheter body 10, and is provided with a protective cap. As shown in Fig. 6, the protective cap includes a securing portion 51 and a protective lumen 52. The diameter of the securing portion 51 is equal to that of the protective lumen 52, and the securing portion 51 is fixedly arranged on the outer surface of the catheter body 10. For example, the securing portion 51 may be fixedly arranged on the outer surface of the catheter body 10 by using various methods such as a hot melt method, an adhesive-based adhesion method, and etc. Optionally, the securing portion 51 is integrally provided with and the protective lumen 52.

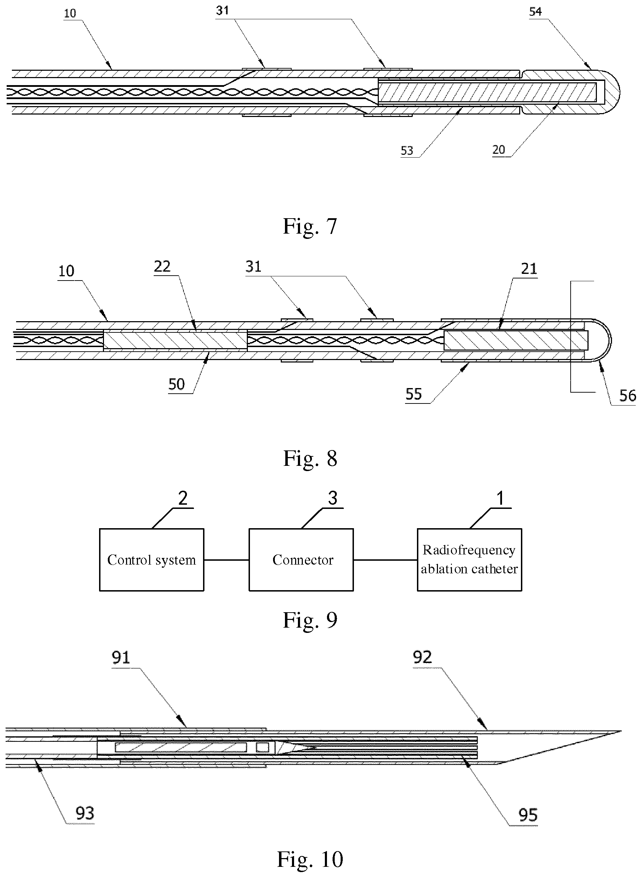

The radiofrequency ablation catheter shown in Fig. 7 includes: a catheter body 10, a localization sensor 20 and ablation electrodes 31. The localization sensor 20 is located at the head portion of the catheter body 10, and is provided with a protective cap. As shown in Fig. 7, the protective cap includes a securing portion 53 and a protective lumen 54. The diameter of the securing portion 53 is smaller than that of the protective lumen 54, and the securing portion 53 is fixedly arranged on the inner surface of the catheter body 10. For example, the securing portion 53 may be fixedly arranged on the inner surface of the catheter body 10 by using various methods such as a hot melt method, an adhesive-based adhesion method, and etc. Optionally, the securing portion 53 is integrally provided with the protective lumen 54.

The radiofrequency ablation catheter shown in Fig. 8 includes: a catheter main body 10, a first localization sensor 21, a second localization sensor 22 and ablation electrodes 31. The first localization sensor 21 and the second localization sensor 22 are separately arranged on two sides of the ablation electrodes 31. The first localization sensor 21 is fixedly arranged at the head portion of the catheter body 10, and is provided with a protective cap. As shown in Fig. 8, the protective cap includes a securing portion 55 and a protective lumen 56. The diameter of the securing portion 55 is equal to that of the protective lumen 56, and the securing portion 55 is fixedly arranged on the outer surface of the catheter body 10. For example, the securing portion 55 may be fixedly arranged on the outer surface of the catheter body 10 by using various methods such as a hot melt method, an adhesive-based adhesion method, and etc. Optionally, the securing portion 55 is integrally provided with the protective lumen 56.

Optionally, the protective caps of all the above-mentioned structures can be electrically connected with a radiofrequency ablation generator to be used as an ablation electrode.

Further optionally, as shown in Fig. 8, the second localization sensor 22, which is arranged at the middle portion of the catheter body 10, is not suitable to be provided with a protective cap, but in order to protect the second localization sensor 22, a protective layer 50 may be arranged on the outer surface of the second localization sensor 22. It should be noted that the second localization sensor 22 also may be provided without a protective layer 50.

On the basis of all the above-mentioned embodiments, in order to use the radiofrequency ablation catheter more flexibly, the radiofrequency ablation catheter also may include: a guidance catheter sheath which is arranged on the outer side of the catheter body and is used for providing a transtracheal pathway for the radiofrequency ablation catheter. Further, the guidance catheter sheath also may be a pre-bent catheter, so that the radiofrequency ablation catheter may enter a correct bronchial branch by rotating the guidance catheter sheath at a position of the bronchial bifucation, which is more flexible and easier to use.

In the embodiments of the present application, the type of the localization sensor is not limited. Optionally, the localization sensor may be an electromagnetic navigation localization sensor, but not limited thereto. For example, a 5 degree-of-freedom electromagnetic navigation localization sensor or a 6 degree-of-freedom electromagnetic navigation localization sensor may be adopted.

In actual use, radiofrequency ablation may possibly cause interference to the localization sensor and affect the localization effect, so that a localization sensor with an electromagnetic shielding layer may be adopted, such as the 5 degree-of-freedom electromagnetic navigation localization sensor, but is not limited thereto. In addition, lead wires which connect the localization sensor with the external control system also may be shielding wires, so as to further reduce the interference in a transmission process of the current signal. The use of the localization sensor with the electromagnetic shielding layer may not only shield the interference caused by the radiofrequency ablation and improve the localization accuracy, but also realize real-time localization in the radiofrequency ablation, prevent the deviation of an ablation position due to the unexpected movement of ablation catheter, thereby improving the accuracy and the efficiency of treatment.

Further, based on the consideration of other factors, such as the localization accuracy, part of the localization sensors may adopt the localization sensors having the electromagnetic shielding layers. As shown in Fig. 4a or Fig. 8, the first localization sensor 21 may be the 6 degree-of-freedom electromagnetic navigation sensor without electromagnetic shielding layer, and the second localization sensor 22 may be the 5 degree-of-freedom electromagnetic navigation sensor with electromagnetic shielding layer. In actual use, the 5 degree-of-freedom and/or 6 degree-of-freedom electromagnetic navigation sensor may be used to locate the radiofrequency ablation catheter to the lesion position. After the ablation is initiated, only the 5 degree-of-freedom electromagnetic navigation sensor is used to locate the radiofrequency ablation catheter. In this way, the localization accuracy may be guaranteed, and the interference caused by the radiofrequency ablation may be also shielded.

It is described here that in Figs. 1 to 8, a straight line which is displayed in the catheter body 10 and is connected with the ablation electrodes 31, and a cross curve which is connected with the localization sensor 20, the first localization sensor 21 or the second localization sensor 22 are lead wires.

The radiofrequency ablation catheter provided by the above-mentioned embodiments of the present application may be applied to a radiofrequency ablation system, and may cooperate with other components in the radiofrequency ablation system to complete the radiofrequency ablation. Fig. 9 is a schematic diagram of a structure of a radiofrequency ablation system provided by another embodiment of the present application. As shown in Fig. 9, the radiofrequency ablation system includes: a radiofrequency ablation catheter 1, a control system 2 and a connector 3. The control system 2 is mainly used for controlling the operation of radiofrequency ablation catheter 1. The radiofrequency ablation catheter 1 is operated under the control of the control system 2. The radiofrequency ablation catheter 1 is connected with the control system 2 through the connector 3. Namely, the radiofrequency ablation catheter 1 is connected with an end of the connector 3, and the control system 2 is connected with the other end of the connector 3. The structure of the radiofrequency ablation catheter 1 is as shown in Figs. 1 to 8, so that no more details will be described here.

Taking the structure of the radiofrequency ablation catheter 1 shown in Fig. 1 as an example, the connection between the radiofrequency ablation catheter 1 and the connector 3 mainly refers to that: the localization sensor 20 and the ablation electrode 31 are respectively connected with the connector 3. Herein, the localization sensor 20 and the ablation electrode 31 may be connected with the connector 3 through lead wires. The embodiments of the present application do not limit an implementation mode of the lead wires. Preferably, these lead wires are connected with the connector 3 through the interior of the catheter body 10.

Preferably, the radiofrequency ablation catheter 1 is connected to the connector 3 in such a manner that: the ablation electrode 31 is connected with the connector 3 through one lead wire, and the localization sensor 20 is connected with the connector 3 through multiple lead wires, specifically depending on the type of the localization sensor 20. For example, if the localization sensor 20 is the 5 degree-of-freedom electromagnetic navigation sensor, two lead wires may be adopted to connect the localization sensor 20 with the connector 3. If the localization sensor 20 is the 6 degree-of-freedom electromagnetic navigation sensor, four lead wires may be used to connect the localization sensor 20 with the connector 3. Optionally, a temperature sensor, if included, may be connected with the connector 3 through two lead wires.

A working principle of the radiofrequency ablation system is briefly described below by taking such a case for example that the radiofrequency ablation system performs the radiofrequency ablation on the basis of a CT (Computed Tomography) and/or a bronchial tree three-dimensional image of a lesion region (such as a lung region).

An initial path planning stage is that:
the control system 2 imports CT image data and/or the bronchial tree three-dimensional graphics of the lesion region, and an initial navigation path from the main trachea to multiple stages of bronchi of the lesion region is drawn on the CT image data and/or the bronchial tree three-dimensional image.

A path navigation stage is that:
the radiofrequency ablation catheter 1 is delivered to the airway through a bronchoscope along the initial navigation path, and a magnetic field generator in the control system 2 continuously generates magnetic fields. The localization sensor in the radiofrequency ablation catheter 1 generates a current signal in response to the magnetic field in the airway and feeds the current signal back to the control system 2 in real time. The control system 2 continuously calculates position coordinates of the radiofrequency ablation catheter 1 according to the current signal fed back by the localization sensor, and continuously guides the radiofrequency ablation catheter to advance along the navigation path until the radiofrequency ablation catheter 1 is guided to the lesion position.

A radiofrequency ablation stage is that:
the radiofrequency ablation generator in the control system 2 provides an ablation solution (mainly including output power, ablation time and the like of the ablation electrode) for the radiofrequency ablation catheter 1, so as to control the radiofrequency ablation catheter 1 to perform radiofrequency ablation. The radiofrequency ablation generator also may adjust the ablation solution according to a treatment situation.

In addition, the control system 2 also may track the position coordinates of the radiofrequency ablation catheter 1 in real time according to the current signal fed back by the localization sensor, and the position of the radiofrequency ablation catheter 1 in the airway is adjusted manually, so that the problem that the inaccurate position of the radiofrequency ablation catheter due to change of lesion positions caused by respiratory movement of the lung or other unexpected movements may be solved, and the radiofrequency ablation is performed more accurately.

In the radiofrequency ablation system provided by the present embodiment, by arranging the localization sensor inside the radiofrequency ablation catheter, the radiofrequency ablation catheter may be accurately located to the lesion position through navigation of the localization sensor, so that the problem of inaccurate position of the radiofrequency ablation catheter placed through a bronchoscope can be solved. In addition, the localization sensor is configured to track the position of the radiofrequency ablation catheter in real time to solve such a problem that the inaccurate position of the radiofrequency ablation catheter due to the change of the lesion positions caused by the respiratory movement of the lung or other accidental movements, which is favorable to improve the therapeutic effect.

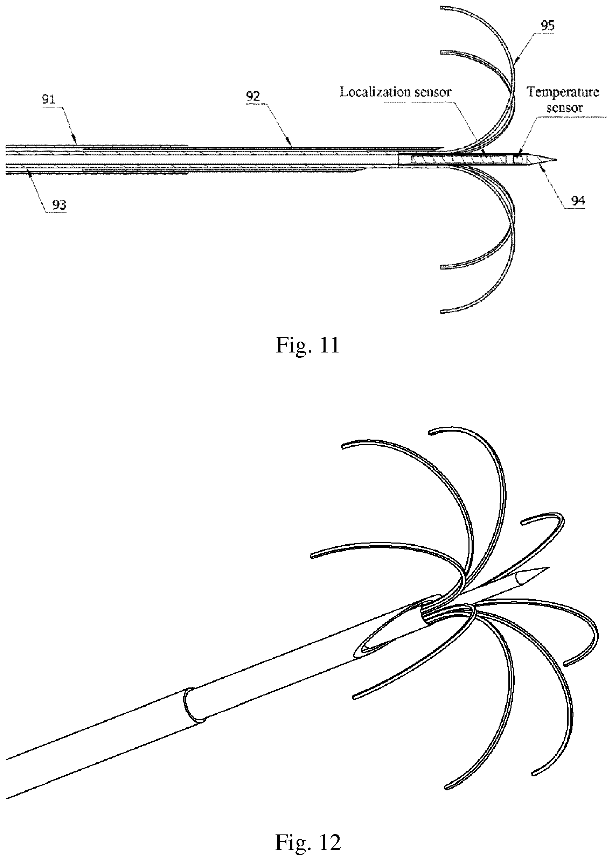

In addition to the above-mentioned radiofrequency ablation electrode and system, the embodiments of the present application further provide an ablation catheter with a localization sensor and an umbrella electrode. The ablation catheter may cooperative with a guidance catheter or a bronchoscope. The structure of the ablation catheter is described below in conjunction with Figs. 10 to 12.
Herein, Fig. 10 is a schematic diagram of a structure of an ablation catheter provided by another embodiment of the present application when an umbrella electrode is in a retracted state. Fig. 11 is a schematic diagram of a structure of an ablation catheter provided by a further embodiment of the present application when an umbrella electrode is in an expanded-out state. Fig. 12 is an overall schematic diagram of a structure of an ablation catheter provided by a further embodiment of the present application.

Referring to Fig. 10 and Fig. 11, the ablation catheter includes: a catheter body 91, a trocar 92, an electrode catheter 93, a center electrode 94 and an umbrella electrode 95.

Herein, the trocar 92 is connected and fixed to the front end of the catheter body 91. The electrode catheter 93 is arranged inside the catheter body 91, and is telescopically movable. In short, the electrode catheter 93 may move forwards and backwards inside the catheter body 91 and the trocar 92, so as to change from the state shown in Fig. 10 to the state shown in Fig. 11 or Fig. 12 for facilitating the ablation.

The catheter body 91 and the electrode catheter 93 should be able to bear an axial tensive force and pressure, and make the change in length as small as possible, so as to ensure that an electrode may be expanded out and retracted smoothly.

Optionally, the catheter body 91 may have a structure of tightly wrapped helical metal wires, or a structure in which braided metal wires are provided inside the catheter, or a structure in which a helical metal wire is provided inside the catheter. Correspondingly, the electrode catheter 93 may have a structure of tightly wrapped helical metal wires, or a structure in which braided metal wires are provided inside the catheter, or a structure in which a helical metal wire is provided inside the catheter. Optionally, the catheter may be made of a multi-layer polymeric material, such as Pebax, TPU, PTFE and the like. The main functions of these structures are the transmission of the axial force and a torque, so as to prevent the catheter body 91 or the electrode catheter 93 from being kinked by an external force, or from being bent during bending. The umbrella electrode 95 is insulated from the metal part of the catheter body 91.

Continuously referring to Fig. 10 and Fig. 11, the center electrode 94 is connected and fixedly arranged at the head portion of the electrode catheter 93. The center electrode 94 has a sharp head portion that may be inserted into a tissue. The center electrode 94 has a hollow structure, in which the localization sensor is disposed. The localization sensor may cooperate with the external control system to acquire a position and a direction of the ablation catheter, thereby ensuring that the center electrode 94 and the umbrella electrode 95 accurately reach the lesion position that is required to be ablated. The localization sensor is based on an electromagnetic navigation principle, and may generate a current signal in response to a localization magnetic field of a space where the localization sensor is located and output the current signal to a control system outside the ablation catheter, so as to enable the control system to determine a position of the ablation catheter.

Continuously referring to Fig. 10 and Fig. 11, the umbrella electrode 95 is fixedly arranged at the head portion of the center electrode 94. The embodiments do not limit a fixed arrangement mode. For example, the umbrella electrode 95 may be welded at the head portion of the center electrode 94. The center electrode 94 and the umbrella electrode 95 may be electrically communicated with the external control system through a lead wire or the electrode catheter 93, so as to allow an ablation current to pass through. In addition, the umbrella electrode 95 is an elastic curved metal needle, and has a sharp tip that may be inserted into the tissue. The umbrella electrode 95 may be expanded out or retracted from the interior of the trocar 92 at the distal end of the catheter body 91.

In an optional implementation, the ablation catheter is a unipolar ablation catheter. The polarity of the umbrella electrode 95 is the same as that of the center electrode 94, and an electrode with the other polarity may be attached to the outer surface of a human body. Continuously referring to Fig. 11, a temperature sensor is further arranged inside the center electrode 94, and is used for monitoring a temperature in an ablation region. Herein, the temperature sensor and the localization sensor are connected with the external control system through lead wires to transmit electric signals.

During use of the above-mentioned ablation catheter, the trocar 92 is firstly inserted into the tissue, and then the electrode catheter 93 is pushed to enable the umbrella electrode 95 and the center electrode 94 to extend out of the inserted tissue.

An ablation catheter with an annular electrode is required to enter an existing channel, and the electrode is required to be in contact with a conductive tissue. The ablation catheter with the umbrella electrode, which is provided by the present embodiment, does not need an existing channel. Firstly, the trocar may be inserted into the tissue at a position close to a target position, so as to arrive at the target position, and then the umbrella electrode therein is pushed out to be further inserted into the tissue for performing the ablation.

According to the ablation catheter with the umbrella electrode, which is provided by the present embodiment, by arranging the localization sensor inside the center electrode, the ablation catheter may be accurately located to a lesion position through navigation of the localization sensor, so that the problem of inaccurate position of the ablation catheter placed by a bronchoscope can be solved. In addition, the localization sensor is used to track the position of the ablation catheter in real time to solve such a problem that the inaccurate position of the ablation catheter due to change of lesion positions caused by respiratory movement of the lung or other accidental movements.

Persons skilled in the art should understand that the embodiments of the present disclosure may provide a method, a system or a computer program product. Therefore, the present disclosure may adopt the form of a complete hardware embodiment, a complete software embodiment, or a software and hardware combined embodiment. In addition, the present disclosure may adopt the form of a computer program product implemented on one or multiple computer available storage media (including, but not limited to, a magnetic disk memory, a Compact Disc Read-Only Memory (CD-ROM), an optical memory and the like) including computer-sensitive program codes.

The present disclosure is described by referring to flowcharts and/or block diagrams of methods, devices (systems) and computer program products according to the embodiments of the present disclosure. It should be understood that each flow and/or each block in the flowcharts and/or the block diagrams and a combination of the flows and/or the blocks in the flowcharts and/or the block diagrams may be implemented by the computer program instructions. These computer program instructions may be provided for a processor of a general computer, a dedicated computer, an embedded processor or other programmable data processing devices to generate a machine, so that an apparatus for achieving functions designated in one or more flows of the flowcharts and/or one or more blocks of the block diagrams is generated via instructions executed by the processor of the computers or the other programmable data processing devices.

These computer program instructions also may be stored in a computer readable memory capable of guiding the computer or other programmable data processing devices to work in a specific manner, so that a manufactured product including an instruction apparatus is generated via the instructions stored in the computer-readable memory, and the instruction apparatus achieves the functions designated in one or multiple flow of flowcharts and/or one or multiple blocks of the block diagrams.

These computer program instructions, which can also be loaded onto the computers or the other programmable data processing devices, enable the computers to implement a series of operation steps on the computers or the other programmable data processing devices; therefore, the instructions executed on the computers or the other programmable data processing devices provide steps of achieving the functions designated in one or multiple flows of the flowcharts and/or one or multiple blocks of the block diagrams.

In a typical configuration, a computing device includes one or multiple Central Processing Units (CPUs), one or multiple input/output interfaces, one or multiple network interfaces and one or multiple internal memories.

The memory may include a non-persistent memory, a Random Access Memory (RAM) and/or a non-volatile memory, etc., such as an ROM or a flash RAM, in a computer-readable medium. The memory is an example of the computer-readable medium.

The computer-readable medium includes persistent, non-persistent, removable media and non-removable media which may realize information storage by any methods or technologies. Information may be computer readable instructions, data structures, modules of programs or other data. Examples of computer storage media include, but are not limited to, a Phase-change Random Access Memory (PRAM), a Static Random Access Memory (SRAM), a Dynamic Random Access Memory (DRAM), other types of RAMs, an ROM, an Electrically Erasable Programmable Read-Only Memory (EEPROM), a flash memory or other memory technologies, a CD-ROM, a Digital Video Disk (DVD) or other optical memories, a magnetic cartridge type magnetic tape, a magnetic tape/disk storage device or other magnetic storage devices or any other non-transmission media, and may be used for storing information accessible by the computing device. The computer readable medium does not include transitory media, such as modulated data signals and carriers, according to definitions herein.

It also should be noted that terms "include", "comprise" or any other variants are meant to cover non-exclusive inclusions, so that a process, method, commodity or device that includes a series of elements not only includes those elements, but also includes other elements which are not definitely listed, or further includes inherent elements of this process, method, commodity or device. Without more restrictions, elements defined by a sentence "includes a/an ..." do not exclude that the process, method, commodity or device that includes the elements still includes other identical elements.

Persons skilled in the art should understand that the embodiments of the present application may provide a method, a system or a computer program product. Therefore, the present application may adopt the form of a complete hardware embodiment, a complete software embodiment, or a software and hardware combined embodiment. In addition, the present application may adopt the form of a computer program product implemented on one or multiple computer-sensitive storage media (including, but not limited to, a magnetic disk memory, a CD-ROM, an optical memory and the like) including computer-sensitive program codes.

The above are merely the embodiments of the present application, but are not intended to limit the present application. Persons skilled in the art can make various changes and modifications to the present application. Any modifications, equivalent replacements, improvements and the like that are made without departing from the spirit and the principle of the present application shall fall within the protection scope defined by the appended claims of the present application.

## Claims

1. A radiofrequency ablation catheter,comprising:
a catheter body;
a localization sensor, fixedly arranged inside the catheter body; and
ablation electrodes, fixedly arranged on an outer surface of the catheter body and used for generating radiofrequency currents, wherein the localization sensor generates a current signal in response to a localization magnetic field of a space where the localization sensor is located, and outputs the current signal to a control system outside the radiofrequency ablation catheter, so as to enable the control system to determine a position of the radiofrequency ablation catheter.

2. The radiofrequency ablation catheter according to claim 1, wherein a water-cooling structure for accommodating electrode cooling liquid is arranged inside the catheter body.

3. The radiofrequency ablation catheter according to claim 2, wherein the catheter body comprises therein at least two lumina along a length direction of the catheter body,
wherein the localization sensor is fixedly arranged in one of the at least two lumina; and the other lumen of the at least two lumina and a gap formed by the other lumen and the lumen with the localization sensor form the water-cooling structure .

4. The radiofrequency ablation catheter according to claim 3, wherein the at least two lumina comprise: a first lumen having an axial line that is collinear with an axial line of the catheter body and a second lumen surrounding the first lumen,
wherein the localization sensor is fixedly arranged in the first lumen; and the second lumen is used as a water inlet channel, and the gap between the second lumen and the first lumen is used as a water outlet channel, so as to form the water-cooling structure.

5. The radiofrequency ablation catheter according to claim 2, wherein a water-cooling pipe with openings at both ends is arranged inside the catheter body,
wherein the water-cooling pipe is used as a water inlet channel, and an outer wall of the water-cooling pipe and an inner wall of the catheter body form a water outlet channel, so as to form the water-cooling structure.

6. The radiofrequency ablation catheter according to claim 5, wherein an end, away from the localization sensor, of the catheter body is provided with a water inlet pipe and a water outlet pipe,
wherein the water inlet pipe is connected with an end, away from the localization sensor, of the water-cooling pipe, and the water outlet pipe is connected with an end, away from the localization sensor, of the water outlet channel.

7. The radiofrequency ablation catheter according to claim 1, wherein the localization sensor comprises: a first localization sensor fixedly arranged at a head portion of the catheter body.

8. The radiofrequency ablation catheter according to claim 7, wherein the localization sensor further comprises: a second localization sensor.

9. The radiofrequency ablation catheter according to claim 1, wherein at least two ablation electrodes are provided and arranged on the outer surface of the catheter body along the length direction of the catheter body in a spacing manner.

10. The radiofrequency ablation catheter according to claim 1, further comprising: a temperature sensor used for detecting temperatures of the ablation electrodes.

11. The radiofrequency ablation catheter according to any one of claims 1 to 10, further comprising:
a protective structure for preventing the localization sensor from being deformed by a force.

12. The radiofrequency ablation catheter according to claim 11, wherein the protective structure comprises:
a protective layer fixedly arranged on the outer surface of the localization sensor, and/or
a protective cap sleeved on the head portion of the localization sensor or the head portion of the catheter body.

13. The radiofrequency ablation catheter according to claim 12, wherein the protective cap comprises: a securing portion for securing the protective cap and a protective lumen for accommodating the head portion of the localization sensor.

14. The radiofrequency ablation catheter according to claim 13, wherein a diameter of the securing portion is equal to a diameter of the protective lumen, and the securing portion is fixedly arranged on the outer surface of the catheter body; or
the diameter of the securing portion is less than the diameter of the protective lumen, and the securing portion is fixedly arranged on the inner surface of the catheter body.

15. The radiofrequency ablation catheter according to claim 12, wherein the protective cap is made of a metal material, and the protective cap is electrically connected with the control system to generate a radiofrequency current under the control of the control system.

16. The radiofrequency ablation catheter according to any one of claims 1 to 10, further comprising:
a guidance catheter sheath arranged on the outer side of the catheter body.

17. The radiofrequency ablation catheter according to claim 16, wherein the guidance catheter sheath is a pre-bent catheter.

18. A radiofrequency ablation system, comprising:
a radiofrequency ablation catheter;
a control system configured for controlling the operation of the radiofrequency ablation catheter; and
a connector configured for connecting the radiofrequency ablation catheter with the control system,
wherein the radiofrequency ablation catheter comprises:
a catheter body;
a localization sensor, fixedly arranged inside the catheter body; and
ablation electrodes, fixedly arranged on an outer surface of the catheter body and used for generating radiofrequency currents,
wherein the localization sensor generates a current signal in response to a localization magnetic field of a space where the localization sensor is located, and outputs the current signal to the control system, to enable the control system to determine a position of the radiofrequency ablation catheter.

19. An ablation catheter, comprising:
a catheter body;
a trocar connected and fixed at a front end of the catheter body;
an electrode catheter, arranged inside the catheter body and telescopically movable; and
a center electrode, connected and fixed at the head portion of the electrode catheter, wherein the head portion of the center electrode is fixedly provided with an umbrella electrode;
the center electrode is of a hollow structure; a localization sensor is arranged inside the center electrode; wherein the localization sensor generates a current signal in response to a localization magnetic field of a space where the localization sensor is located, and outputs the current signal to a control system outside the ablation catheter, so as to enable the control system to determine a position of the ablation catheter.

20. The ablation catheter according to claim 19, wherein the umbrella electrode has the same polarity as the center electrode, and a temperature sensor is further arranged inside the center electrode.

21. The ablation catheter according to claim 19 or 20, wherein the catheter body has a structure of tightly wrapped helical metal wires, or a structure in which braided metal wires are provided inside the catheter, or a structure in which a helical metal wire is provided inside the catheter; and
the electrode catheter has a structure of tightly wrapped helical metal wires, or a structure in which braided metal wires are provided inside the catheter, or a structure in which a helical metal wire is provided inside the catheter.
